(19) Europäisches Patentamt
European Patent Office
Office européen des brevets

(11) EP 0 860 165 B1

(12) FASCICULE DE BREVET EUROPEEN

(45) Date de publication et mention de la délivrance du brevet: **03.05.2000 Bulletin 2000/18**

(51) Int Cl.[7]: **A61K 7/42**

(21) Numéro de dépôt: **98400113.1**

(22) Date de dépôt: **21.01.1998**

# (54) Compositions cosmétiques pour la photoprotection de la peau et/ou des cheveux à base d'un mélange synergique de filtres

Kosmetische Mittel zum Lichtschutz der Haut und/oder der Haare auf Basis einer synergistischen Mischung von Filtern

Sun-protection cosmetic compositions for skin and/or hair based on a synergic mixture of filters

(84) Etats contractants désignés:
**AT BE CH DE DK ES FI FR GB GR IE IT LI LU MC NL PT SE**

(30) Priorité: **24.02.1997 FR 9702162**

(43) Date de publication de la demande:
**26.08.1998 Bulletin 1998/35**

(73) Titulaire: **L'OREAL**
**75008 Paris (FR)**

(72) Inventeur: **Hansenne, Isabelle**
**75017 Paris (FR)**

(74) Mandataire: **Miszputen, Laurent**
**L'OREAL-DPI**
**6 rue Bertrand Sincholle**
**92585 Clichy Cédex (FR)**

(56) Documents cités:
**WO-A-94/06404**

- **GESSNER G. HAWLEY: 'The condensed chemical dictionary', 1981, VAN NOSTRAND REINHOLD COMPANY, NEW YORK, TENTH EDITION * page 988 ***

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 860 165 B1

Printed by Jouve, 75001 PARIS (FR)

## Description

La présente invention concerne de nouvelles compositions cosmétiques et/ou dermatologiques plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association entre un premier filtre particulier, à savoir un dérivé sulfonique particulier du benzimidazole avec au moins un deuxième filtre particulier convenablement sélectionné au sein des silicones benzotriazoles.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire (SPF) s'exprimant mathématiquement par le rapport du temps d'irradiation nécessaire pour atteindre le seuil érythématogène avec le filtre UV au temps nécessaire pour atteindre le seuil érythématogène sans filtre UV).

Or, à la suite d'importantes recherches menées dans le domaine de la photoprotection évoqué ci-dessus, la Demanderesse a découvert, de façon inattendue et surprenante, que la combinaison, dans des proportions comprises dans des limites bien déterminées, de deux filtres solaires particuliers et déja connus en soi dans l'état de l'art, permettait, du fait d'un effet de synergie remarquable, d'obtenir des compositions antisolaires présentant des indices de protection nettement améliorés, et en tous cas largement supérieurs à ceux qui peuvent être obtenus soit avec l'un ou l'autre des filtres utilisé seul, soit encore avec des associations contenant simultanément les deux filtres mais dans des rapports sortant du domaine de l'invention.

Ainsi, conformément à l'un des objets de la présente invention, il est maintenant proposé de nouvelles compositions cosmétiques et/ou dermatologiques, en particulier antisolaires, caractérisées par le fait qu'elles comprennent, dans un support cosmétiquement acceptable,

- i) à titre de premier filtre, au moins un dérivé silicié à fonction benzotriazole comprenant au moins une unité de formule (1) suivante :

$$O_{(3-a)/2}\ Si\ (R)_a\ -\ A \qquad (1)$$

dans laquelle :

- R représente un radical alkyle en $C_1$-$C_{10}$ éventuellement halogéné ou un radical phényle ou un radical triméthylsilyloxy,
- a est un nombre entier choisi entre 0 et 3 inclusivement,
- et le symbole A désigne un radical monovalent lié directement à un atome de silicium, et qui répond à la formule (2) suivante :

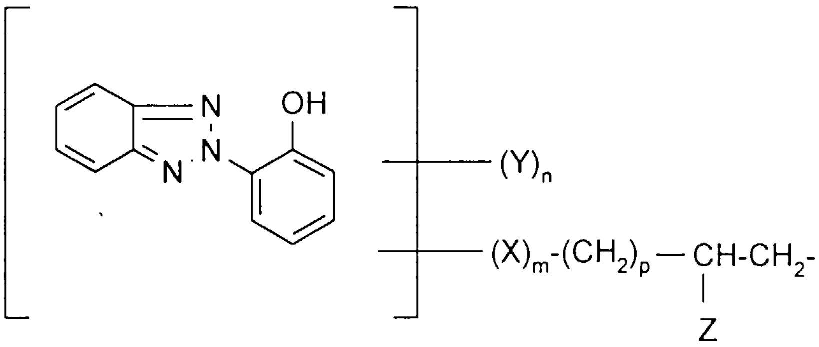

(2)

dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement,

- ii) à titre de deuxième filtre, au moins un dérivé sulfonique de benzimidazole C répondant à la formule (3) suivante :

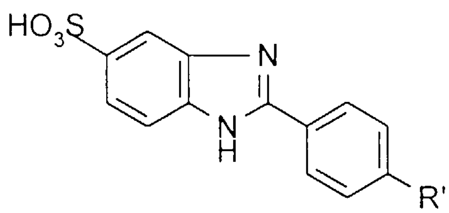

(3)

dans laquelle R' désigne un atome d'hydrogène, un radical alkyle ou alcoxy, linéaire ou ramifié, en $C_1$-$C_8$ ou un radical de formule (4) suivante :

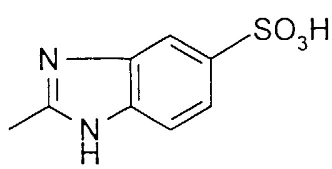

(4)

lesdits premier et deuxième filtres étant présents dans lesdites compositions dans un rapport molaire (A/C) approprié pour l'obtention d'une amélioration synergique du SPF.

La présente invention a également pour objet l'utilisation de telles compositions comme, ou pour la fabrication de, compositions cosmétiques et/ou dermatologiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

D'autres caractéristiques, aspects et avantages de la présente invention apparaîtront à la lecture de la description détaillée qui va suivre.

Les dérivés siliciés utilisés dans la présente invention sont des silanes ou des siloxanes à fonction benzotriazole comprenant au moins une unité de formule (1) suivante :

$$O_{(3-a)/2}\ Si\ (R)_a\ -\ A \qquad (1)$$

dans laquelle :

- R représente un radical alkyle en $C_1$-$C_{10}$ éventuellement halogéné ou un radical phényle ou un radical triméthylsilyloxy,
- a est un nombre entier choisi entre 0 et 3 inclusivement,
- et le symbole A désigne un radical monovalent lié directement à un atome de silicium, et qui répond à la formule (2) suivante :

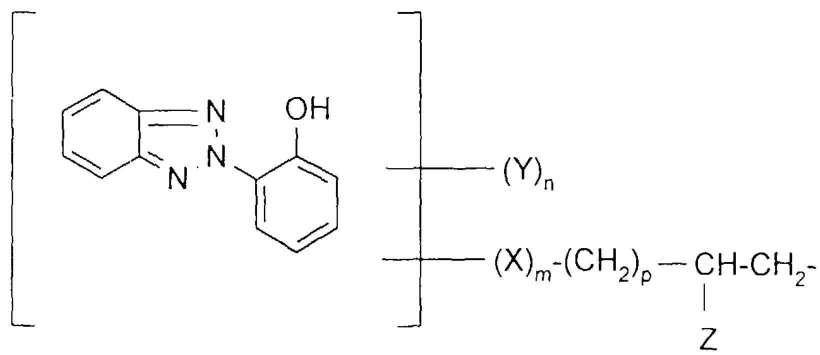

(2)

dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement.

Ces composés sont notamment décrits dans la demande de brevet EP-A-711778 au nom de la Demanderesse ainsi que dans la demande WO 94/06404 également au nom de la Demanderesse.

De préférence, les dérivés siliciés utilisés dans le cadre de la présente invention appartiennent à la famille générale des silicones benzotriazoles qui est décrite notamment dans WO 94/06404. Une famille de silicones benzotriazoles convenant particulièrement bien à la réalisation de la présente invention est celle regroupant les composés répondant aux formules (5) ou (6) suivantes :

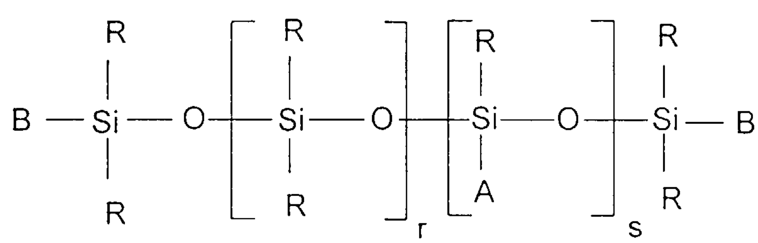

(5)

ou

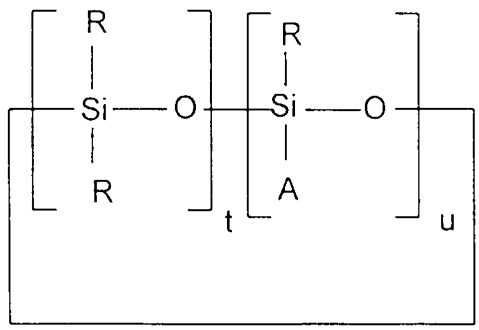

(6)

dans lesquelles :

- R, identiques ou différents, sont choisis parmi les radicaux alkyles en $C_1$-$C_{10}$, phényle, trifluoro-3,3,3 propyle et triméthylsilyloxy, au moins 80% en nombre des radicaux R étant méthyle,
- B, identiques ou différents sont choisis parmi les radicaux R et le radical A,
- r est un nombre entier compris entre 0 et 50 inclusivement, et s est un nombre entier compris entre 0 et 20 inclusivement, et si s = 0, au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A répond à la formule (2) ci-dessus.

Comme cela ressort de la formule (2) donnée ci-dessus, l'accrochage du chaînon $-(X)_m-(CH_2)_p-CH(Z)-CH_2-$ sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazole :

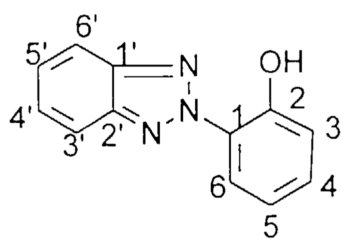

De préférence, cet accrochage se fait en position 3, 4, 5 (noyau aromatique portant la fonction hydroxy) ou 4' (noyau benzénique adjacent le cycle triazolé), et encore plus préférentiellement en position 3, 4 ou 5. Dans une forme préférée de réalisation de l'invention, l'accrochage se fait en position 3.

De même, l'accrochage du ou des motifs substituants Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage du motif Y se fait en position 5.

Dans les formules (5) et (6) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R préférés selon l'invention sont les radicaux méthyle, éthyle, propyle, n-butyle, n-octyle et éthyl-2 hexyle. Encore plus préférentiellement, les radicaux R sont tous des radicaux méthyle.

Parmi les composés de formules (5) ou (6) ci-dessus, on préfère mettre en oeuvre ceux répondant à la formule (5), c'est-à-dire des diorganosiloxanes à chaîne courte linéaire.

Parmi les composés de formules (5) ci-dessus, on préfère mettre en oeuvre ceux pour lesquels les radicaux B sont tous les deux des radicaux R.

Parmi les diorganosiloxanes linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :

- B est un radical R,

- R est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, ter.-butyle ou alcoxy en $C_1$-$C_4$,
- Z est hydrogène ou méthyle,
- m=0, ou [m=1 et X=O]
- p est égal à 1.

Une famille de silicones benzotriazoles convenant particulièrement bien à l'invention est celle définie par la formule générale (7) suivante :

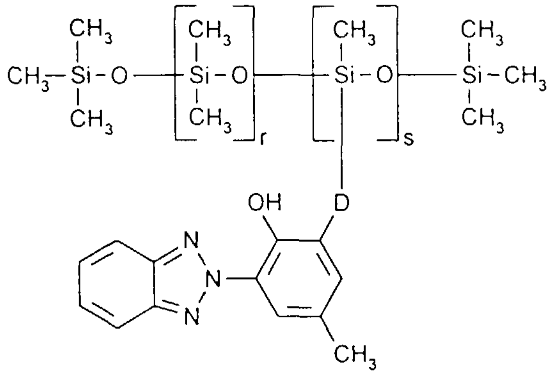

(7)

avec $0 \leq r \leq 10$,

$1 \leq s \leq 10$, et où D représente le radical divalent :

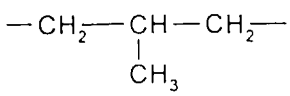

Dans une forme particulièrement préférée de réalisation de l'invention, la silicone benzotriazole est le composé (appelé composé (a) dans la suite du texte) répondant à la formule suivante :

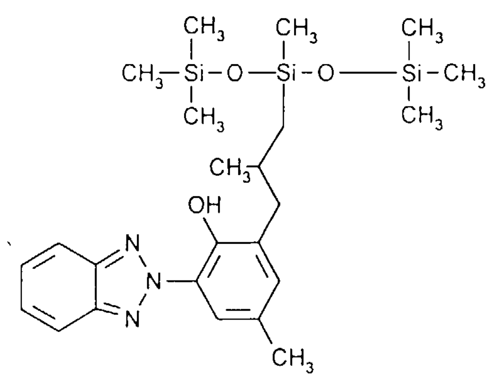

composé (a)

Des procédés convenant à la préparation des produits de formule (1), (5), (6) et (7) ci-dessus sont notamment décrits dans les brevets américains US 3,220,972, US 3,697,473, US 4,340,709, US 4,316,033, US 4,328,346 et dans les demandes de brevet EP-A-0 392 883 et EP-A-0 742 003.

Le dérivé silicié à fonction benzotriazole peut être présent dans les compositions selon l'invention à des teneurs comprises entre 0,1 et 20%, de préférence entre 0,2 et 15%, en poids, toujours par rapport au poids total de la composition.

Les dérivés sulfoniques de benzimidazole (composé C) utilisés dans la présente invention sont des composés hydrosolubles connus pour leur excellent pouvoir photoprotecteur dans le domaine des rayonnements UV-B et répondant à la formule (3) suivante :

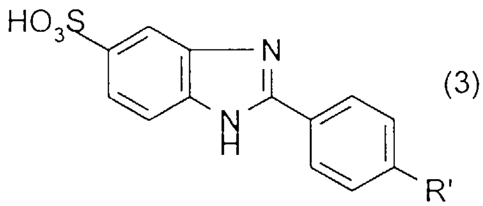

(3)

dans laquelle R' désigne un atome d'hydrogène, un radical alkyle ou alcoxy, linéaire ou ramifié, en $C_1$-$C_8$ ou un radical de formule (4) suivante :

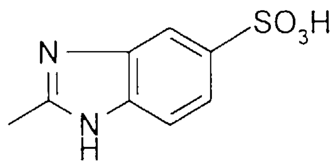

(4)

Un dérivé sulfonique de benzimidazole convenant particulièrement bien aux compositions selon l'invention est l'acide 2-phénylbenzimidazole 5-sulfonique vendu sous la dénomination commerciale « EUSOLEX 232 » par la société MERCK qui répond à la formule suivante :

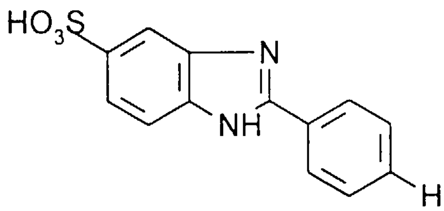

Le dérivé sulfonique de benzimidazole peut être présent dans les compositions selon l'invention à une teneur comprise entre 0,1 et 10%, de préférence entre 0,1 et 5%, en poids par rapport au poids total de la composition.

D'un point de vue pratique, les deux filtres ci-dessus, à savoir le dérivé silicié à motif benzotriazole et le dérivé sulfonique de benzimidazole, sont bien entendu de préférence tous deux présents dans la composition finale dans des proportions respectives choisies de manière telle que l'effet de synergie, au niveau du facteur de protection solaire conféré par l'association résultante, soit optimal.

Ces proportions peuvent varier en fonction du nombre de motif(s) benzotriazole porté(s) par le dérivé silicié à fonction benzotriazole. Ainsi, de préférence, dans la présente invention le rapport molaire [(motif benzotriazole du premier filtre)/dérivé sulfonique de benzimidazole], c'est-à-dire le rapport molaire (A/C) peut aller de 1:20 à 10:3, de préférence de 1:10 à 5:2, de préférence encore de 2:5 à 3:5.

Ainsi, dans le cas particulier où le premier filtre selon la présente invention est le composé siliconé (a) de formule (7) ci-dessus et le deuxième filtre selon la présente invention est le dérivé sulfonique de benzimidazole vendu sous la dénomination commerciale « Eusolex 232 », le rapport pondéral [(premier filtre)/(deuxième filtre)] peut varier de 1:10 à 6:1, de préférence de 1:4 à 4:1. De préférence encore, ce rapport pondéral est de 1:1.

Enfin, toujours selon un mode préféré de réalisation de la présente invention, les compositions selon l'invention sont des émulsions de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les deux filtres mentionnés ci-avant. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des

nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones, les $\alpha$-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-$\alpha$-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, en particulier l'effet de synergie, attachées intrinsèquement à l'association binaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un autre objet de la présente invention réside dans un procédé de

traitement cosmétique de la peau ou des cheveux destiné à les protéger contre les effets des rayons UV consistant à appliquer sur ceux-ci une quantité efficace d'une composition cosmétique telle que définie ci-dessus.

**EXEMPLE 1**

On a préparé diverses formulations antisolaires se présentant sous la forme d'une émulsion de type huile-dans-eau et contenant (les quantités sont exprimées en % de poids par rapport au poids total de la composition) :

Phase A1 :

- composé (a) y % (silicone benzotriazole)
- mélange de stéarate glycérylique et de stéarate PEG-100 vendu sous la dénomination « Arlacel 165 » par ICI 1,5 %
- acide stéarique vendu sous la dénomination « Stéarine TP » par Stéarinerie Dubois 2 ,75 %
- alcool cétylique vendu sous la dénomination « Lorol C16 » par Henkel 0,5 %
- benzoate d'alkyle en $C_{12}$-$C_{15}$ vendu sous la dénomination « Finsolv TN » par Finetex 15 %
- conservateurs qs

Phase A2 :

- triéthanolamine 0,45 %

Phase B:

- acide 2-phénylbenzimidazole 5 sulfonique vendu sous la dénomination commerciale « Eusolex 232 » par Merck x%
- cétyl phosphate de potassium vendu sous la dénomination « Amphisol K » par Givaudan-Roure 1 %

Phase C :

- acide polyacrylique réticulé vendu sous la dénomination « Carbopol 980 » par Goodrich 0,3 %

Phase D:

- triéthanolamine qs

Phase E:

- hydratant 5 %
- conservateur qs
- eau qs 100%

On a réalisé chacune de ces émulsions de la manière suivante: les phases A1 et A2 ont été préalablement chauffées et homogénéisées sous agitation à 80°C. La phase E a été introduite dans la cuve de fabrication et chauffée à 80°C sous agitation. Puis on a dispersé les phases B et C dans la phase E pendant 30 minutes. Sous forte agitation, le mélange (A1 + A2) a été ajouté sur la phase aqueuse (E + B + C). On a agité pendant 15 minutes. On a ensuite refroidi ce mélange jusqu'à 50°C et on a ajouté la phase D. On a refroidi le tout jusqu'à 25°C.

Pour chacune de ces formulations, on a ensuite déterminé le facteur de protection solaire (SPF) qui lui était attaché. Celui-ci a été déterminé en utilisant la méthode *in vitro* décrite par B.L. DIFFEY et al. dans J. Soc. Cosmet. Chem. 40-127-133 (1989) ; cette méthode consiste à déterminer les facteurs de protection monochromatiques tous les 5 nm dans une gamme de longueurs d'onde de 290 à 400 nm et à calculer à partir de ceux-ci le facteur de protection solaire selon une équation mathématique donnée.

Les compositions des différentes formulations étudiées et les résultats en facteur de protection solaire moyen (moyenne sur trois essais) obtenus sont rassemblés dans le tableau (I) donné ci-dessous.

Tableau (I)

| Formules comparatives A à F : | | | | | | |
|---|---|---|---|---|---|---|
| Filtre | Formule A | Formule B | Formule C | Formule D | Formule E | Formule F |
| x (%) | 1 | 0 | 2,5 | 0 | 4 | 0 |
| y (%) | 0 | 4 | 0 | 2,5 | 0 | 1 |
| SPF | 3,7 | 6,8 | 5,2 | 4,5 | 8,5 | 2,4 |

Tableau (II) :

| Formules selon l'invention G, H et J : | | | |
|---|---|---|---|
| Filtre | Formule G | Formule H | Formule J |
| X (%) | 1 | 2,5 | 4 |
| Y (%) | 4 | 2,5 | 1 |
| Rapport pondéral y/x | 4 | 1 | 0,25 |
| Rapport molaire (benzotriazole/benzimidazole) | 2,183 | 0,546 | 0,136 |
| **SPF** moyen | 11,2 | 17,8 | 13,2 |

Ainsi, pour une concentration globale en filtres de 5 % en poids, on obtient :

SPF(formule A) + SPF (formule B) = 10,5　　SPF(formule G) = 11,2

SPF(formule C) + SPF (formule D) = 9,7　　SPF(formule H) = 17,8

SPF(formule E) + SPF (formule F) = 10,9　　SPF(formule J) = 13,2

Ces résultats démontrent clairement l'effet de synergie spectaculaire obtenu avec les compositions G, H et J conformes à l'invention.

## Revendications

**1.** Composition cosmétique et/ou dermatologique, caractérisée par le fait qu'elle comprend, dans un support cosmétiquement acceptable,

- i) à titre de premier filtre, au moins un dérivé silicié à fonction benzotriazole comprenant au moins une unité de formule (1 ) suivante :

$$O_{(3a)/2}\ Si\ (R)_a - A \qquad (1)$$

dans laquelle :

- R représente un radical alkyle en $C_1$-$C_{10}$ éventuellement halogéné ou un radical phényle ou un radical triméthylsilyloxy,
- a est un nombre entier choisi entre 0 et 3 inclusivement,
- et le symbole A désigne un radical monovalent lié directement à un atome de silicium, et qui répond à la

formule (2) suivante :

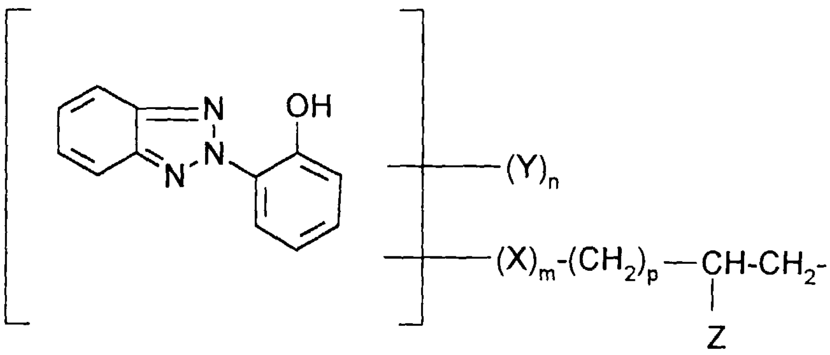

(2)

dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement,

- ii) à titre de deuxième filtre, au moins un dérivé sulfonique de benzimidazole C répondant à la formule (3) suivante :

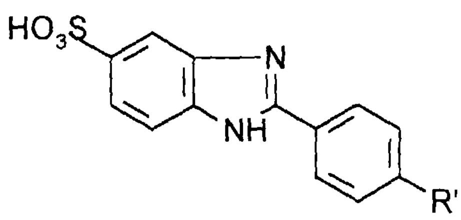

(3)

dans laquelle R' désigne un atome d'hydrogène, un radical alkyle ou alcoxy, linéaire ou ramifié, en C1-C8 ou un radical de formule (4) suivante :

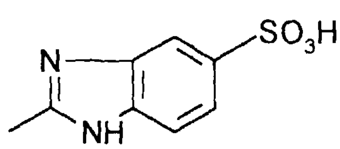

(4)

lesdits premier et deuxième filtres étant présents dans lesdites compositions dans un rapport molaire (A/C )

allant de 1:20 à 10:3.

2. Composition selon la revendication 1, caractérisée par le fait que le rapport molaire (A/C) va de 1:10 à 5:2 et plus préférentiellement de 2:5 à 3:5.

3. Composition selon la revendication 1 ou 2, caractérisée par le fait que le dérivé silicié à fonction benzotriazole répond à l'une des formules (5) ou (6) suivantes :

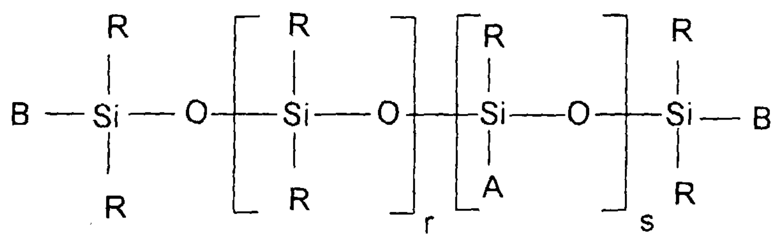

(5)

ou

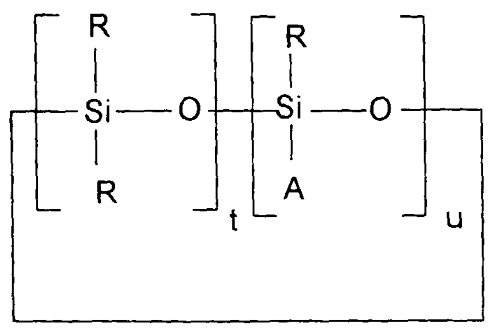

(6)

dans lesquelles :

- R, identiques ou différents, sont choisis parmi les radicaux alkyles en $C_1$-$C_{10}$, phényle, trifluoro-3,3,3 propyle et triméthylsilyloxy, au moins 80% en nombre des radicaux R étant méthyle,
- B, identiques ou différents sont choisis parmi les radicaux R et le radical A,
- r est un nombre entier compris entre 0 et 50 inclusivement, et s est un nombre entier compris entre 0 et 20 inclusivement, et si s = 0, au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A répond à la formule (2) définie dans la revendication 1.

4. Composition selon la revendication 3, caractérisée par le fait que la silicone benzotriazole répond à la formule (7) suivante :

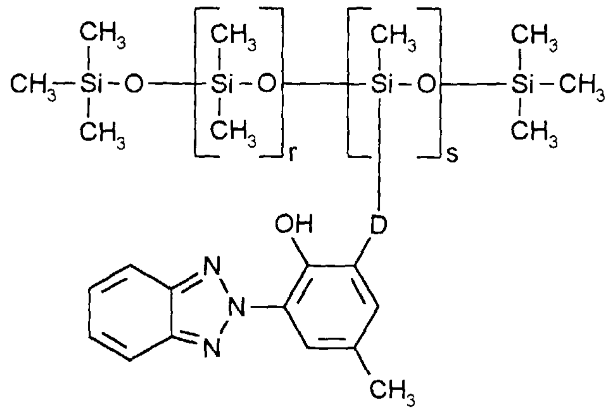

(7)

avec $0 \leq r \leq 10$,
$1 \leq s \leq 10$, et où D représente le radical divalent :

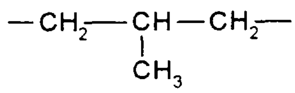

**5.** Composition selon l'une quelconque des revendications précédentes, caractérisée par le fait que le dérivé sulfonique de benzimidazole est l'acide 2-phénylbenzimidazole 5-sulfonique.

**6.** Composition selon la revendication 5, caractérisée par le fait que le dérivé silicié à fonction benzotriazole répond à la formule suivante :

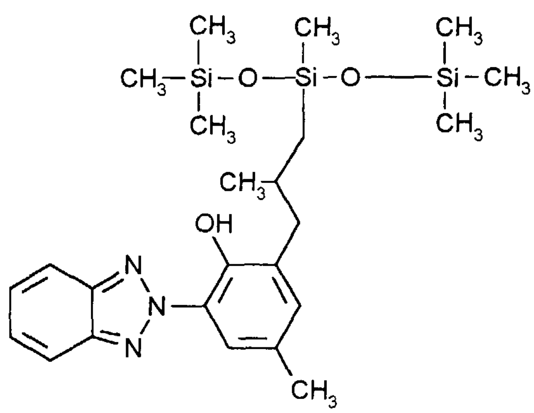

et en ce que le rapport pondéral [(premier filtre)/(deuxième filtre)] va de 1:10 à 6:1, de préférence de 1:4 à 4:1 et de préférence encore est égal à 1.

**7.** Composition selon l'une quelconque des revendications précédentes, caractérisée par le fait que le dérivé silicié à fonction benzotriazole est présent à une teneur comprise entre 0,1 et 20% en poids, de préférence entre 0,2 et 15 % en poids, par rapport au poids total de la composition.

**8.** Composition selon l'une quelconque des revendications précédentes, caractérisée par le fait que le dérivé sulfonique de benzimidazole est présent à une teneur comprise entre 0,1 et 10%, de préférence entre 0,1 et 5%, en

poids par rapport au poids total de la composition.

**9.** Composition selon l'une quelconque des revendications précédentes, caractérisée par le fait qu'elle comprend en outre un ou plusieurs filtres organiques complémentaires actifs dans l'UV-A et/ou UV-B, hydrophiles ou lipophiles.

**10.** Composition selon l'une quelconque des revendications précédentes, caractérisée par le fait qu'elle comprend en outre au moins un agent de bronzage et/ou de brunissage artificiel de la peau.

**11.** Utilisation d'une composition définie à l'une quelconque des revendications précédentes comme, ou pour la fabrication de, compositions cosmétiques pour la protection de la peau et/ou des cheveux contre le rayonnement ultraviolet, en particulier le rayonnement solaire.

**12.** Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 10.

**Patentansprüche**

**1.** Kosmetische und/oder dermatologische Zusammensetzung, dadurch gekennzeichnet, daß sie in einem kosmetisch akzeptablen Träger enthält:

- i) als erstes Filter mindestens ein Siliciumderivat mit Benzotriazolgruppe, das mindestens eine Einheit der folgenden Formel (1) aufweist:

$$O_{(3-a)/2}Si\,(R)_a\text{-}A \qquad (1),$$

worin bedeuten:

- R eine $C_{1-10}$-Alkylgruppe, die gegebenenfalls halogeniert ist, eine Phenylgruppe oder eine Trimethylsilyloxygruppe,
- a 0 oder eine ganze Zahl im Bereich von 1 bis 3, und
- A eine einwertige Gruppe, die direkt an ein Siliciumatom gebunden ist und der folgenden Formel (2) entspricht:

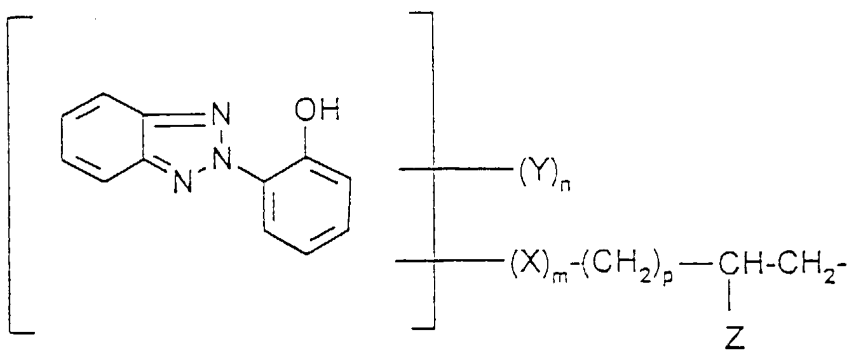
(2),

worin:
- die Gruppen Y, die identisch oder voneinander verschieden sind, unter den $C_{1-8}$-Alkylgruppen, Halogenen und $C_{1-4}$-Alkoxygruppen ausgewählt sind, wobei im letzten Fall zwei angrenzende Gruppen Y an dem gleichen aromatischen Ring auch gemeinsam eine Alkylidendioxygruppe bilden können, worin die Alkylidengruppe 1 bis 2 Kohlenstoffatome aufweist,
- X O oder NH bedeutet,
- Z Wasserstoff oder eine $C_{1-4}$-Alkylgruppe bedeutet,
- n 0 oder eine ganze Zahl im Bereich von 1 bis 3 ist,

- m 0 oder 1 ist, und
- p eine ganze Zahl im Bereich von 1 bis 10 bedeutet, und

- ii) als zweites Filter mindestens ein Sulfonsäurederivat von Benzimidazol C der folgenden Formel (3) :

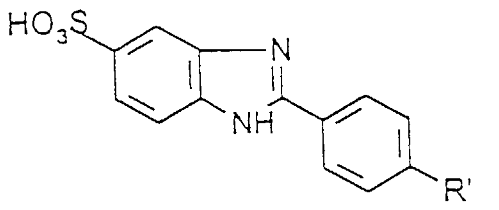

(3),

worin R' ein Wasserstoffatom, eine geradkettige oder verzweigte Alkyl- oder Alkoxygruppe mit 1 bis 8 Kohlenstoffatomen oder eine Gruppe der folgenden Formel (4) bedeutet:

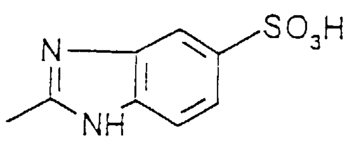

(4),

wobei das erste Filter und das zweite Filter in den Zusammensetzungen in einem Molverhältnis (A/C) im Bereich von 1:20 bis 10:3 vorliegen.

2. Zusammensetzung nach Anspruch 1, dadurch gekennzeichnet, daß das Molverhältnis (A/C) im Bereich von 1:10 bis 5:2 und vorzugsweise im Bereich von 2:5 bis 3:5 liegt.

3. Zusammensetzung nach Anspruch 1 oder 2, dadurch gekennzeichnet, daß das Siliciumderivat mit Benzotriazolgruppe einer der folgenden Formeln (5) oder (6) entspricht:

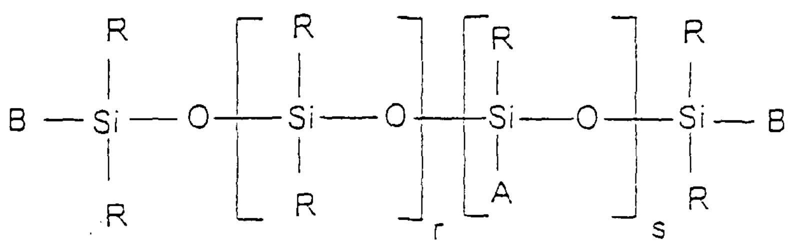

(5)

oder

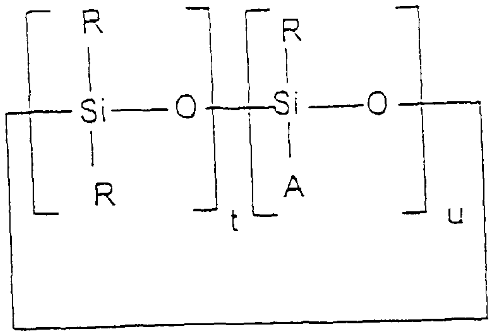

(6),

worin:

- die Gruppen R, die identisch oder voneinander verschieden sind, unter den $C_{1-10}$-Alkylgruppen, Phenyl, 3,3,3-Trifluorpropyl und Trimethylsilyloxy ausgewählt sind, wobei mindestens 80 % der Anzahl der Gruppen R Methyl bedeutet,
- die Gruppen B, die identisch oder voneinander verschieden sind, unter den Gruppen R und der Gruppe A ausgewählt sind,
- r 0 oder eine ganze Zahl im Bereich von 1 bis 50 und s 0 oder eine ganze Zahl im Bereich von 1 bis 20 bedeutet, und mindestens eine der beiden Gruppen B A bedeutet, wenn s 0 ist,
- u eine ganze Zahl im Bereich von 1 bis 6 und t 0 oder eine ganze Zahl im Bereich von 1 bis 10 ist, mit der Maßgabe, daß t + u 3 bedeutet oder darüber liegt, und
- die Gruppe A der Formel (2) nach Anspruch 1 entspricht.

**4.** Zusammensetzung nach Anspruch 3, dadurch gekennzeichnet, daß das Benzotriazolsilicon der folgenden Formel (7) entspricht:

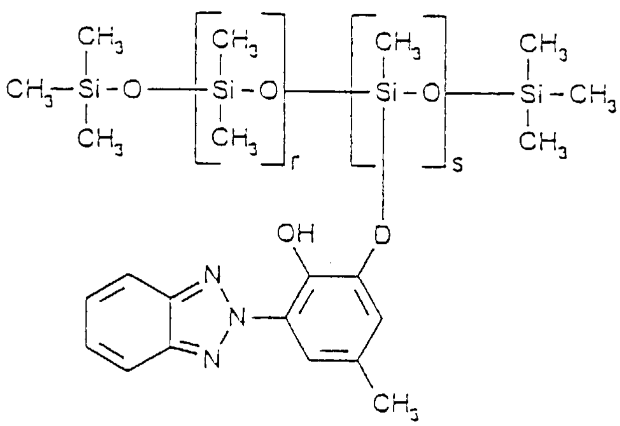

(7)

worin bedeuten:
$0 \leq r \leq 10$,
$1 \leq s \leq 10$, und D die zweiwertige Gruppe:

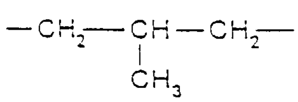

**5.** Zusammensetzung nach einem der vorhergehenden Ansprüche, dadurch gekennzeichnet, daß das Benzimidazolsulfonsäurederivat die-2-Phenyl-benzimidaziol-5-sulfonsäure ist.

**6.** Zusammensetzung nach Anspruch 5, dadurch gekennzeichnet, daß das Siliciumderivat mit Benzotriazolgruppe der folgenden Formel entspricht:

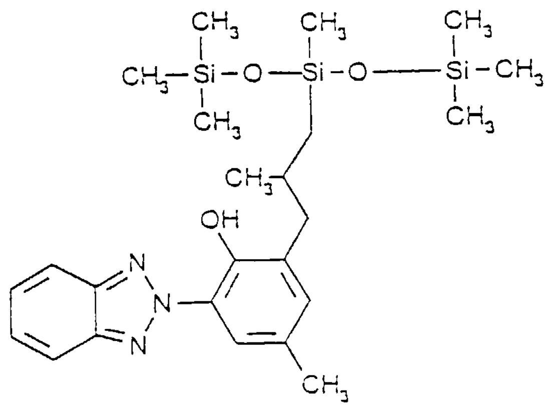

**7.** Zusammensetzung nach einem der vorhergehenden Ansprüche, dadurch gekennzeichnet, daß das Siliciumderivat mit Benzotriazolgruppe in einem Mengenanteil im Bereich von 0,1 bis 20 Gew.-% und vorzugsweise im Bereich von 0,2 bis 15 Gew.-%, bezogen auf das Gesamtgewicht der Zusammensetzung, vorliegt.

**8.** Zusammensetzung nach einem der vorhergehenden Ansprüche, dadurch gekennzeichnet, daß das Benzimidazolsulfonsäurederivat in einem Mengenanteil im Bereich von 0,1 bis 10 Gew.-% und vorzugsweise im Bereich von 0,1 bis 5 Gew.-%, bezogen auf das Gesamtgewicht der Zusammensetzung, vorliegt.

**9.** Zusammensetzung nach einem der vorhergehenden Ansprüche, dadurch gekennzeichnet, daß sie ferner ein oder mehrere zusätzliche, im UV-A- und/oder UV-B-Bereich wirksame, hydrophile oder lipophile, organische Filter enthält.

**10.** Zusammensetzung nach einem der vorhergehenden Ansprüche, dadurch gekennzeichnet, daß sie ferner mindestens Bräunungsmittel und/oder Mittel zur künstlichen Bräunung der Haut enthält.

**11.** Verwendung einer Zusammensetzung nach einem der vorhergehenden Ansprüche als kosmetische Zusammensetzung zum Schutz der Haut und/oder der Haare gegen UV-Strahlung und insbesondere gegen Sonnenlicht oder zur Herstellung dieser Zusammensetzung.

**12.** Kosmetisches Verfahren zum Schutz der Haut und/oder der Haare gegen UV-Strahlung und insbesondere gegen Sonnenlicht, dadurch gekennzeichnet, daß es darin besteht, eine wirksame Menge mindestens einer Zusammensetzung nach einem der Ansprüche 1 bis 10 auf die Haut und/oder die Haare aufzutragen.

## Claims

**1.** Cosmetic and/or dermatological composition, characterized in that it comprises, in a cosmetically acceptable support,

- i) as first screening agent, at least one silicon derivative containing a benzotriazole function, comprising at least one unit of formula (1) below:

$$O_{(3-a)/2}Si(R)_a\text{-}A \qquad (1)$$

in which:

- R represents an optionally halogenated $C_1$-$C_{10}$ alkyl radical or a phenyl radical or a trimethylsilyloxy radical,

- a is an integer chosen between 0 and 3 inclusive,

  - and the symbol A denotes a monovalent radical linked directly to a silicon atom, and which corresponds to formula (2) below:

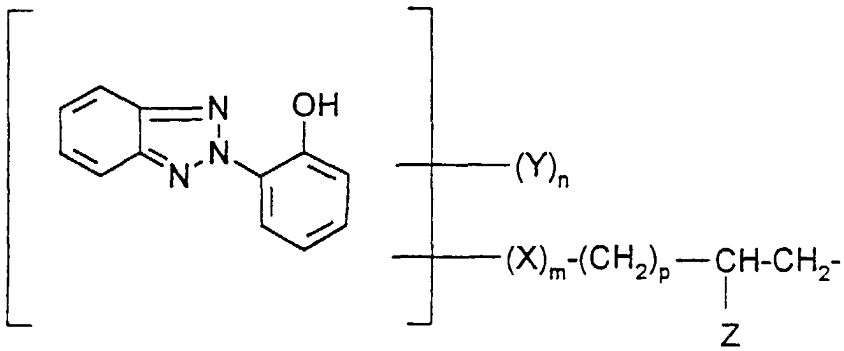

(2)

in which:

  - Y, which may be identical or different, are chosen from $C_1$-$C_8$ alkyl radicals, halogens and $C_1$-$C_4$ alkoxy radicals, it being understood that, in the latter case, two adjacent groups Y on the same aromatic nucleus can together form an alkylidenedioxy group in which the alkylidene group contains 1 or 2 carbon atoms,
  - X represents O or NH,
  - Z represents hydrogen or a $C_1$-$C_4$ alkyl radical,
  - n is an integer between 0 and 3 inclusive,
  - m is 0 or 1,
  - p represents an integer between 1 and 10 inclusive,

- ii) as second screening agent, at least one sulphonic derivative of benzimidazole C corresponding to formula (3) below:

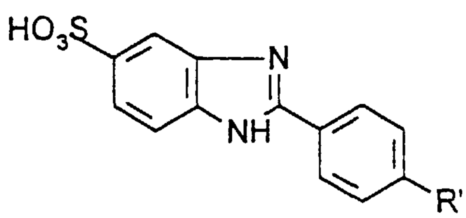

(3)

in which R' denotes a hydrogen atom, a linear or branched $C_1$-$C_8$ alkyl or alkoxy radical or a radical of formula (4) below:

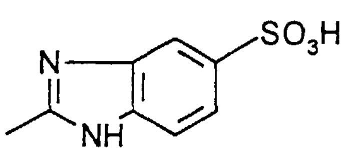

(4)

the said first and second screening agents being present in the said compositions in a molar ratio (A/C) ranging from 1:20 to 10:3.

**2.** Composition according to Claim 1, characterized in that the molar ratio (A/C) ranges from 1:10 to 5:2 and more preferably from 2:5 to 3:5.

**3.** Composition according to Claim 1 or 2, characterized in that the silicon derivative containing a benzotriazole function corresponds to either of the formulae (5) and (6) below:

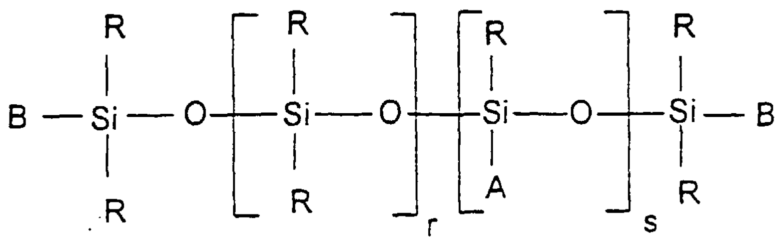

(5)

and

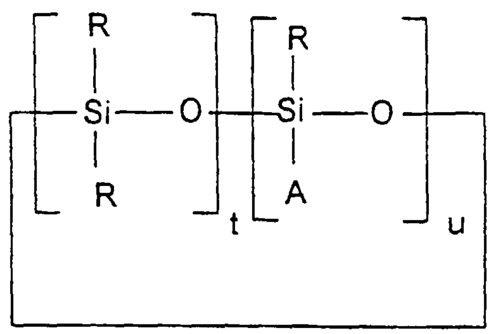

(6)

in which:

- R, which may be identical or different, are chosen from $C_1$-$C_{10}$ alkyl, phenyl, 3,3,3-trifluoropropyl and trimethylsilyloxy radicals, at least 80%, in numerical terms, of the radicals R being methyl,
- B, which may be identical or different, are chosen from the radicals R and the radical A,
- r is an integer between 0 and 50 inclusive, and s is an integer between 0 and 20 inclusive, and, if s = 0, at least one of the two symbols B denotes A,
- u is an integer between 1 and 6 inclusive, and t is an integer between 0 and 10 inclusive, it being understood that t + u is greater than or equal to 3, and
- the symbol A corresponds to formula (2) defined in Claim 1.

**4.** Composition according to Claim 3, characterized in that the benzotriazole silicone corresponds to formula (7) below:

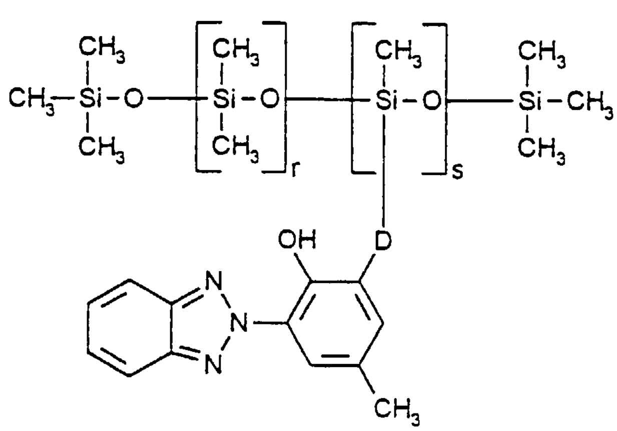

(7)

with $0 \leq r \leq 10$,
$1 \leq s \leq 10$, and in which D represents the divalent radical:

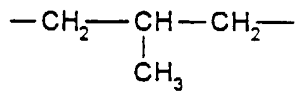

**5.** Composition according to any one of the preceding claims, characterized in that the sulphonic derivative of benzimidazole is 2-phenylbenzimidazole-5-sulphonic acid.

**6.** Composition according to Claim 5, characterized in that the silicon derivative containing a benzotriazole function corresponds to the following formula:

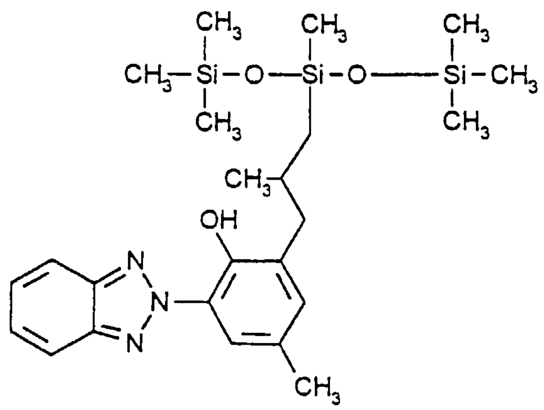

and in that the weight ratio [(first screening agent)/(second screening agent)] ranges from 1:10 to 6:1, preferably from 1:4 to 4:1 and even more preferably is equal to 1.

**7.** Composition according to any one of the preceding claims, characterized in that the silicon derivative containing a benzotriazole function is present in a content of between 0.1 and 20% by weight, preferably between 0.2 and 15% by weight, relative to the total weight of the composition.

**8.** Composition according to any one of the preceding claims, characterized in that the sulphonic derivative of benzimidazole is present in a content of between 0.1 and 10%, preferably between 0.1 and 5%, by weight relative to the total weight of the composition.

**9.** Composition according to any one of the preceding claims, characterized in that it also comprises one or more additional hydrophilic or lipophilic organic screening agents which are active in the UV-A and/or UV-B range.

**10.** Composition according to any one of the preceding claims, characterized in that it also comprises at least one agent for artificially tanning and/or browning the skin.

**11.** Use of a composition defined in any one of the preceding claims as, or for the manufacture of, cosmetic compositions for protecting the skin and/or the hair against ultraviolet radiation, in particular solar radiation.

**12.** Cosmetic treatment process for protecting the skin and/or the hair against ultraviolet radiation, in particular solar radiation, characterized in that it consists in applying an effective amount of a composition as defined in any one of Claims 1 to 10 to the skin and/or the hair.